# FASCICULE DE BREVET EUROPEEN

(11) **EP 4 304 471 B1**
(45) Date de publication et mention de la délivrance du brevet: **05.11.2025**
(21) Numéro de dépôt: 22710497.3
(22) Date de dépôt: 16.02.2022
(51) Int. Cl.: A61B 5/06, G01V 3/08, G01V 3/165, G16H 30/40, G16H 50/20, G16H 50/50, A61B 5/00

(54) **PROCÉDÉ D'IMAGERIE D'UN IMPLANT IMPLANTÉ**
VERFAHREN ZUR ABBILDUNG EINES IMPLANTIERTEN IMPLANTATS
METHOD FOR IMAGING AN IMPLANTED IMPLANT

(30) Priorité: 08.03.2021 FR 2102213
(43) Date de publication de la demande: 17.01.2024
(73) Titulaire: Bonetag, 66000 Perpignan (FR); Université de Montpellier, 34090 Montpellier (FR); Centre National de la Recherche Scientifique, 75016 Paris (FR)
(72) Inventeur: NAUDI, Stéphane, 66000 PERPIGNAN (FR); VENA, Arnaud, 34270 SAINT-MATHIEU-DE-TRÉVIERS (FR); DUTRIEUX, Sylvain, 34270 LE TRIADOU (FR); SORLI, Brice, 34530 MONTAGNAC (FR)
(74) Mandataire: IPAZ
(86) Numéro de dépôt international: PCT/EP2022/053752
(87) Numéro de publication internationale: WO 2022/189106

(56) Documents cités:
- WO-A2-2007/130510
- CN-A- 103 528 495
- CN-A- 111 407 279
- US-A1- 2006 016 452
- US-A1- 2007 238 984
- US-A1- 2016 302 692
- US-A1- 2020 197 103
- TROBEC R ET AL: "Metal implant localizers: frontiers and diagnostic feasibility", JOURNAL OF MEDICAL ENGINEERING & TECHNOLOGY, INFORMA HEALTHCARE, GB, vol. 20, no. 3, 1 May 1996 (1996-05-01), pages 134 - 140, XP008165952, ISSN: 0309-1902, DOI: 10.3109/03091909609008392

## Description

### Domaine technique

La présente invention concerne un procédé d'imagerie d'un implant, comprenant au moins une partie métallique, implanté dans un corps, en particulier d'un être vivant. Elle concerne également un dispositif d'imagerie mettant en œuvre ce procédé d'imagerie.

Le domaine de l'invention est celui des procédés et dispositifs d'imagerie dans le domaine médical, par exemple ceux qui permettent d'évaluer la qualité de pose et le bon fonctionnement d'un implant et/ou d'une prothèse implanté(e) dans un corps.

### Etat de la technique

On connaît des procédés d'imagerie d'éléments implantés dans le corps humain. Ces dispositifs sont fonctionnels mais posent différents problèmes, notamment de :
- difficulté de mise en œuvre car ces procédés utilisent des dispositifs volumineux et nécessitent souvent l'utilisation d'un dispositif d'imagerie mobile pour imager l'ensemble de la zone d'intérêt,
- de sécurité car ils nécessitent l'utilisation d'une imagerie à rayons X pouvant être contraignante car elle implique de faire attention à la dose de rayons X utilisée pour l'imagerie de l'être vivant,
- de temps car de tels procédés nécessitent l'acquisition de plusieurs images dans le temps et dans l'espace pour pouvoir imager l'ensemble de la zone d'intérêt, et
- de coût car de tels procédés sont onéreux, ce qui est souvent lié aux moyens utilisés pour les réaliser et les contraintes qui en découlent, tels que les temps de calcul et de réalisation nécessaires à leurs mises en œuvre.

Les documents WO2007/130510A2, US2016/302692A1 et US2007/238984A1 divulguent des techniques de localisation d'implant. En particulier le document US 2016/302692 A1 divulgue un dispositif communicant portatif permettant de détecter et prédire la position d'un dispositif médical implanté de manière non-invasive. La position prédite pour le dispositif médical implanté peut être affichée sur une interface utilisateur au moyen d'un indice visuel superposé à une représentation corporelle schématique.

Un but de l'invention est de remédier à au moins un des inconvénients précités.

Un autre but de l'invention est de proposer un procédé d'imagerie d'un implant implanté dans un corps plus facile à mettre en œuvre.

Un autre but de l'invention est de proposer un dispositif d'imagerie plus sécurisé.

Un autre but de l'invention est de proposer un procédé d'imagerie plus rapide.

Un autre but de l'invention est de proposer un procédé d'imagerie moins coûteux.

### Exposé de l'invention

L'invention est définie par les revendications jointes.

L'invention permet d'atteindre au moins un des buts précités par un procédé d'imagerie, d'un implant implanté dans un corps, comprenant au moins une itération d'une phase de caractérisation comprenant les étapes suivantes :
- mesure, par au moins un capteur, dit de mesure, disposé hors dudit corps, fournissant au moins un signal électrique, dit signal mesuré, relatif audit implant,
- détermination, par une unité de traitement, d'au moins une donnée relative au positionnement dudit implant en fonction dudit signal mesuré et d'un modèle préalablement établi reliant l'au moins un signal mesuré à l'au moins une donnée relative au positionnement, et
- fourniture, par une unité de traitement, d'une image, dite mesurée, à partir de l'au moins une donnée positionnement et d'un modèle visuel relatif audit implant.

Un tel procédé est mis en œuvre par l'utilisation d'un ou plusieurs capteurs de mesure et d'une unité de traitement, ce qui dénote sa facilité de mise en œuvre.

Le procédé selon l'invention est aussi facile et plus rapide à mettre en œuvre car il ne nécessite pas de prise d'image de l'implant lui-même implanté dans le corps mais de signaux électriques relatifs à l'implant ce qui est plus simple à réaliser et à traiter comparé à la prise d'image. En outre, comme il utilise un signal mesuré, et un modèle visuel relatif audit implant déjà enregistré, ledit procédé d'imagerie limite le temps de calcul nécessaire à la fourniture de l'image mesurée et les coûts de mise en œuvre associés à ces temps de calcul.

Autrement dit, une fois que l'implant est implanté dans le corps, l'image mesurée qui est fournie par le procédé est une image visuelle soit de cet implant soit d'un autre implant analogue qui a été préalablement mémorisée dans une base de données. Ladite étape de mesure est en effet mise en œuvre non pas pour produire en tant que telle cette image mesurée mais pour déterminer un positionnement de l'implant afin d'adapter le modèle visuel préalablement établi en fonction de la disposition effective de l'implant dans le corps.

Le procédé selon l'invention est aussi plus sécurisé car il s'appuie sur une mesure ne nécessitant aucun rayon X, ce qui lui confère une simplicité d'utilisation et le rend plus sécurisé et moins nocif pour la santé du corps dans lequel est implanté l'implant.

Le procédé selon l'invention ne nécessite pas la combinaison de plusieurs images pour afficher l'image mesurée. Par conséquent, le dispositif selon l'invention est plus rapide.

Comme le procédé est plus simple à mettre en œuvre, plus sécurisé et plus rapide, les coûts associés sont donc moins onéreux.

L'au moins une donnée relative au positionnement peut comprendre au moins une donnée de position et/ou au moins une donnée d'inclinaison et/ou au moins une donnée d'orientation et/ou au moins un état (plié et/ou allongé) dudit implant. Dans le contexte de l'invention, l'au moins une donnée de positionnement comprend au moins une donnée d'orientation de l'implant.

De préférence, l'au moins une donnée relative au positionnement peut comprendre trois données de position et trois données d'orientation dudit implant.

Si l'implant est en plusieurs parties ou comprend plusieurs parties, l'au moins une donnée de positionnement peut comprendre trois données de position et trois données d'orientation, par exemple trois positions spatiales et trois angles d'inclinaison, par partie de l'implant.

L'étape de fourniture comprend une étape de modification ou d'adaptation du modèle visuel relatif audit implant à partir de l'au moins une donnée de positionnement, comme par exemple un changement d'orientation du modèle visuel ou d'une partie du modèle visuel.

L'étape de mesure peut mesurer au moins un signal magnétique pour fournir l'au moins un signal mesuré.

Le modèle visuel peut être une image réelle dudit implant, c'est-à-dire une image à partir de laquelle les dimensions réelles de l'implant peuvent être extraites.

Le modèle visuel peut être une image préenregistrée dudit implant, par exemple une image fournie par le constructeur dudit implant. Dans une autre variante, le modèle visuel peut être généré par imagerie scanner.

Le terme corps est assimilé au corps d'un objet, ou un robot, ou d'un être vivant tel qu'un animal ou un humain.

L'implant imagé peut être un implant monobloc, tel que par exemple un implant fémoral ou tibial.

L'implant imagé peut être constitué de plusieurs parties, par exemple un implant de hanche ou d'épaule dans lequel deux parties s'emboitent, ou un implant de cheville composé en trois parties.

Alternativement, l'implant peut comprendre au moins deux parties dont au moins une est mobile par rapport au reste de l'implant, tel que par exemple un implant de genou composé, par exemple, d'un implant fémoral et tibial, un implant de coude, etc.

Le modèle visuel relatif audit implant peut être une image tridimensionnelle dudit implant.

L'image mesurée peut être une image tridimensionnelle.

Ainsi, le procédé selon l'invention peut fournir une image en trois dimensions dudit implant tout en étant facile à mettre en œuvre, rapide, sécurisé et moins couteux.

L'étape de mesure du procédé selon l'invention peut comprendre une mesure, par au moins un capteur inertiel, d'au moins une donnée inertielle relative au corps, et en ce que l'étape de fourniture de l'image mesurée comprend un ajustement de l'orientation du modèle visuel.

La mesure de la donnée inertielle permet de positionner ledit implant dans l'espace, notamment de lui attribuer au moins une orientation, par exemple un angle d'inclinaison exact tel qu'il se trouve lors de l'étape de mesure dans le corps dans lequel il est implanté.

Le procédé selon l'invention peut calculer à partir de la donnée inertielle :
- la position et l'orientation, par exemple au moins un angle d'inclinaison, de préférence trois angles, dans l'espace en trois dimensions de l'implant, et/ou
- la position et l'orientation, par exemple au moins un angle d'inclinaison, de préférence trois angles, de l'implant par rapport à un référentiel choisi, par exemple le sol.

Le procédé selon l'invention peut par cette mesure connaître le mouvement relatif de la partie du corps comprenant l'implant, par rapport à un référentiel choisi, par exemple le sol. Le procédé selon l'invention peut donc déterminer si le patient est allongé ou debout pendant l'examen et également reconstruire une image mesurée latéralisée, c'est-à-dire capable de donner une vue de droite ou de gauche de la partie du corps comprenant l'implant.

Le procédé selon l'invention peut comprendre plusieurs itérations de la phase de caractérisation fournissant chacune une image mesurée, ledit procédé comprenant en outre une génération d'une vidéo à partir desdites images mesurées.

Le procédé selon l'invention permet ainsi de générer une image vidéo de l'implant et donc de reconstruire la dynamique d'un mouvement d'une partie du corps sur lequel l'implant est implanté. Une imagerie dynamique dudit implant peut donc être enregistrée.

Une telle vidéo permet de suivre les mouvements de l'implant et en particulier des différentes parties de l'implant lorsque celui-ci comporte des parties en mouvement les unes par rapport aux autres.

L'au moins un signal mesuré peut comprendre :
- une impédance électrique, dite individuelle ou indépendante, pour chaque capteur de mesure, ou
- une impédance électrique, dite impédance mutuelle, pour chaque capteur de mesure couplé à au moins un autre capteur de mesure, ou
- une tension électrique.

Les capteurs de mesure utilisés pour mesurer les signaux mesurés peuvent être issus de composants disponibles sur le marché ou à l'inverse, être faits entièrement sur-mesure, par exemple à partir de pistes de cuivre.

Le capteur de mesure peut être agencé pour mesurer au moins un signal magnétique et fournir, en sortie, l'au moins un signal mesuré. Le signal en sortie du capteur de mesure peut être proportionnel au signal à l'entrée du capteur de mesure.

Le modèle visuel dudit implant peut être enregistré sur une base de données.

Pour au moins un implant, le modèle visuel relatif audit implant peut être mémorisé en association avec un identifiant de l'implant. Dans ce cas, le procédé selon l'invention peut comprendre une étape d'identification de l'implant en vue de retrouver ledit modèle visuel relatif audit implant.

L'implant implanté dans le corps peut être identifié par le patient ou la personne dans le corps duquel l'implant est implanté, en fournissant une donnée d'identification de l'implant.

Alternativement, le procédé selon l'invention peut comprendre une étape d'identification de l'implant par lecture d'une donnée d'identification fournie par ledit implant.

Par exemple, l'étape d'identification peut comprendre une étape de lecture d'une donnée d'identification mémorisée dans un dispositif électronique intégré dans l'implant. Un tel dispositif électronique peut comprendre une radio étiquette comprenant une antenne associée à une puce électronique contenant au moins une donnée d'identification de l'implant, et optionnellement des données complémentaires.

La donnée d'identification mémorisée dans un tel dispositif électronique peut être lue par un lecteur électronique. Le lecteur électronique peut être externe audit implant.

La lecture de la donnée d'identification d'implant peut être similaire à celle décrite dans le brevet français 3 017 227 A1.

Suivant encore une autre alternative, le modèle visuel relatif audit implant peut être directement récupéré sur le patient, ce qui permet au procédé selon l'invention d'être mis en œuvre par n'importe quel praticien qui possède les éléments permettant de mettre en œuvre ledit procédé.

Le modèle visuel du procédé selon l'invention peut être enregistré sur une base de données, ledit procédé pouvant comprendre une étape d'échange de données entre ladite unité de traitement et ladite base de données pour réaliser l'étape de fourniture.

Le procédé selon l'invention peut aussi récupérer le modèle visuel sur une base de données.

Toute base de données mentionnée peut être locale, comme enregistrée sur un serveur local, ou externe en étant enregistrée sur un serveur externe. La base de données peut être connectée avec l'unité de traitement du procédé selon l'invention. La connexion avec ce serveur local ou externe peut être filaire, ou sans fil via une connexion internet telle que le WIFI, le réseau cellulaire, de téléphonie mobile ou GSM par exemple le réseau 4G ou 5G. Elle peut être faite par une connexion réseau et/ou internet. Le serveur local ou externe peut être apte à communiquer avec l'unité de traitement utilisée dans le procédé selon l'invention.

Le procédé selon l'invention peut comprendre une recherche sur une base de données du modèle visuel à partir de la donnée d'identification d'implant.

La donnée d'identification d'implant peut être déterminée à partir du dossier patient. Le dossier patient peut être retrouvé par un spécialiste en charge de la personne comprenant l'implant et/ou à partir du dossier patient enregistré localement ou sur une base de données en réseau.

La donnée d'identification de l'implant peut permettre de retrouver d'autres informations, par exemple le fabriquant, le modèle de l'implant ainsi que ses dimensions.

Ainsi, dans le procédé selon l'invention, la géométrie exacte de l'implant est connue au préalable. La géométrie exacte de l'implant peut donc être obtenue à partir du dossier patient enregistré sur une base de données ou à partir du dispositif électronique combiné avec le lecteur de radio-étiquette.

Le procédé selon l'invention peut comprendre, préalablement à la première itération de la phase de caractérisation, une phase préalable réalisée lorsque ledit implant est en dehors dudit corps, ladite phase préalable comprenant au moins une itération des étapes suivantes de :
- mesure, par au moins un capteur, dit de mesure, disposé hors dudit corps, fournissant au moins un signal électrique, dit de référence, et
- mémorisation de l'au moins un signal de référence en association avec au moins une donnée de positionnement, dite donnée de positionnement de référence, dudit implant.

Ainsi, suivant le procédé selon l'invention, chaque signal de référence mesuré est associé à au moins une donnée de positionnement de référence dudit implant.

La phase préalable permet d'enregistrer des positionnements dudit implant qui va être implanté afin de connaître des orientations, des inclinaisons, des positions, des états exacts dudit implant en relation avec au moins un signal de référence. Par état exact, on entend par exemple un état plié ou allongé dudit implant.

La phase préalable peut ensuite être utilisée par l'unité de traitement dans l'étape de détermination afin de déterminer l'au moins une donnée relative au positionnement dudit implant implanté dans ledit corps. Dans ce cas, la donnée de positionnement de référence peut être associée à l'au moins une donnée de positionnement utilisée par l'unité de traitement dans l'étape de fourniture de l'image mesurée.

L'au moins un capteur de mesure utilisé dans la phase préalable peut être similaire à, identique, ou différent de l'au moins un capteur de mesure utilisé lors de l'étape de mesure.

L'au moins une donnée de positionnement de référence dudit implant comprend au moins une donnée d'inclinaison et/ou au moins une donnée de positionnement.

De préférence, le signal de référence peut être mémorisé dans l'étape de mémorisation avec trois données d'inclinaison et trois données de positionnement relatif audit implant.

De préférence, l'implant est positionné sur un support mobile dans la phase préalable. Le support mobile comprend au moins un degré de liberté en rotation, et/ou en translation. Cela permet d'enregistrer différentes données de positionnement de l'implant associées avec au moins un signal de référence.

De préférence, le support mobile dudit implant comprend trois degrés de liberté en rotation et trois degrés de liberté en translation.

De cette manière, toutes les positions et orientations de l'implant peuvent être enregistrées. Les données enregistrées dans la phase préalable sont précises et complètes.

L'implant est de préférence imagé sur tous ses angles de vue.

Le modèle préalablement établi peut comprendre :
- un réseau neuronal supervisé entrainé avec une base de données et prenant en entrée l'au moins un signal mesuré, ladite base de données reliant un signal mesuré avec une donnée de positionnement, ou
- une table préenregistrée, par exemple un abaque, associant au moins un signal électrique à au moins une donnée relative à un positionnement dudit implant, ou
- une relation mathématique reliant l'au moins un signal mesuré à l'au moins une donnée relative au positionnement.

L'apprentissage du réseau neuronal peut être effectué avec des données préalablement enregistrées dans la phase préalable, par exemple celles enregistrées dans la base de données suite à l'étape de mémorisation. Par exemple, l'apprentissage peut être effectué à partir de 100 signaux de référence associés à avec trois données de positions et trois données d'inclinaison de référence et 20 signaux mesurés inconnus, c'est-à-dire non associés à au moins une donnée de positionnement. Bien entendu le réseau neuronal supervisé peut être testé avec des données tests, par exemple 20 signaux mesurés inconnus.

La table peut être enregistrée dans l'étape préalable dudit procédé. Dans le cas l'unité de traitement peut être agencée pour calculer la corrélation entre les signaux électriques enregistrés dans la table et ceux mesurés à l'étape de mesure. L'unité de traitement peut être agencée pour sélectionner l'au moins une donnée de position associée au signal électrique de la table ayant la plus forte corrélation avec l'au moins un signal mesuré.

La relation mathématique peut être une matrice ou une fonction de transfert reliant l'au moins un signal mesuré à l'au moins une donnée de positionnement.

Selon un autre aspect de l'invention, il est proposé un dispositif d'imagerie d'un implant implanté dans un corps, comprenant des moyens agencés pour mettre en œuvre le procédé d'imagerie selon l'invention.

En particulier, ledit dispositif d'imagerie selon l'invention comprend
- au moins un capteur, dit de mesure, disposé hors dudit corps et agencé pour fournir au moins un signal électrique, dit mesuré, relatif audit implant,
- une unité de traitement agencée pour :
   ∘ déterminer au moins une donnée relative au positionnement dudit implant en fonction du signal mesuré et d'un modèle préalablement établi reliant l'au moins un signal mesuré à l'au moins une donnée relative au positionnement, et
   ∘ fournir, une image, dite mesurée, à partir de l'au moins une donnée de positionnement et d'un modèle visuel relatif audit implant.

L'au moins un capteur peut être de préférence agencé pour mesurer au moins un signal magnétique et fournir au moins un signal électrique.

Le dispositif d'imagerie selon l'invention nécessite d'utiliser un ou plusieurs capteurs de mesure et d'une unité de traitement, ce qui dénote sa facilité de mise en œuvre.

Le dispositif selon l'invention ne nécessite pas d'utiliser un système d'imagerie à rayon X, ce qui le rend plus simple à mettre en œuvre, plus sécurisé et moins onéreux.

De manière générale, le dispositif d'imagerie selon l'invention permet d'obtenir des avantages similaires à ceux développés pour le procédé d'imagerie selon l'invention car il est agencé pour le mettre en œuvre.

L'au moins un capteur de mesure peut être, de préférence, un capteur inductif.

L'unité de traitement peut comprendre un premier module de calcul configuré pour déterminer l'au moins une donnée de positionnement en fonction de l'au moins un signal mesuré et du modèle préalablement établi.

L'unité de traitement peut comprendre un deuxième module de calcul pour fournir l'image mesurée en fonction de l'au moins une donnée de positionnement et du modèle visuel de l'implant. De manière optionnelle, le deuxième module peut prendre en compte la donnée inertielle afin d'ajuster l'orientation dudit modèle visuel.

L'unité de traitement peut comprendre un module de communication pour échanger des données avec une base de données.

Le dispositif d'imagerie selon l'invention peut comprendre au moins un capteur inertiel agencé pour mesurer au moins une donnée inertielle relative au corps, ladite donnée inertielle pouvant être utilisée par l'unité de traitement pour ajuster l'orientation du modèle visuel.

Au moins un capteur inertiel peut comprendre un capteur de contact en contact avec une surface dudit corps.

Ainsi, le dispositif selon l'invention peut connaître le mouvement relatif de la partie du corps comprenant l'implant dans l'espace vis-à-vis d'un référentiel choisi, tel que le sol.

L'au moins un capteur de mesure réalisant la mesure dudit signal mesuré peut comprendre, ou être, un capteur de champ proche.

L'au moins un capteur électrique peut être inductif.

Ainsi, le dispositif d'imagerie selon l'invention peut utiliser des capteurs de mesure sans contact avec ledit corps.

De manière générale, l'au moins un capteur de mesure peut comprendre un capteur de proximité, détectant de préférence des matériaux conducteurs (par exemple, du métal), ou des matériaux magnétiques. Dans le contexte de l'invention, l'au moins un capteur est un capteur de proximité configuré pour détecter l'au moins une partie métallique de l'implant.

L'au moins un capteur de mesure du dispositif selon l'invention peut comprendre un capteur sans contact.

L'au moins un capteur de mesure et/ou inertiel peut être mobile.

Le dispositif d'imagerie selon l'invention peut comprendre plusieurs capteurs de mesure formant une matrice de capteurs, ladite matrice étant agencée pour entourer au moins une partie du corps comprenant ledit implant.

Ainsi, le dispositif selon l'invention peut comprendre plusieurs capteurs de mesure mesurant le signal mesuré. Ces plusieurs capteurs de mesure sont agencés selon un agencement qui leur permet d'entourer la zone du corps comprenant l'implant (zone d'intérêt). Une cartographie de la zone peut être obtenue par un tel agencement. La mesure du signal mesuré est donc réalisée selon une multitude d'angles de captation. Un tel agencement des capteurs de mesure permet de déterminer de manière précise la position d'un ou plusieurs implants ou des zones particulières de l'implant.

Le dispositif selon l'invention peut comprendre un capteur de mesure utilisé comme récepteur. Par exemple, l'implant peut être agencé pour envoyer au moins un signal qui pourra ensuite être reçu par le capteur de mesure utilisé comme récepteur. Dans une autre variante de dispositif selon l'invention, le capteur de mesure utilisé comme récepteur peut recevoir un signal réfléchi par l'implant.

Le dispositif selon l'invention peut comprendre plusieurs capteurs de mesure comprenant au moins un capteur mesure utilisé comme récepteur et un capteur de mesure utilisé comme émetteur. Par exemple, l'un des capteurs de mesure utilisé comme émetteur peut être agencé pour envoyer un signal à destination de l'implant. Ce signal peut être de préférence magnétique. A réception du signal, l'implant peut passer d'un mode veille à un mode actif et réémettre ainsi un signal qui pourra être reçu par le capteur de mesure utilisé comme récepteur.

L'implant peut donc réfléchir une partie du signal, de préférence une partie du champ magnétique, incident émis par l'au moins un capteur de mesure utilisé comme émetteur. Ce champ magnétique réfléchi peut être réceptionné par un capteur de mesure récepteur.

Dans une variante, le capteur utilisé comme récepteur et l'au moins un capteur utilisé comme émetteur peuvent être compris dans un même capteur de mesure (i.e. un même ensemble).

Le capteur de mesure utilisé comme émetteur peut comprendre une inductance, par exemple une bobine, agencée pour envoyer un signal, étant de préférence à destination de l'implant. Le signal peut être modifié suivant la position et/ou l'orientation et/ou le mouvement de l'implant.

Le signal émis par le capteur de mesure utilisé comme émetteur peut être un signal électrique, ou de préférence magnétique, par exemple inductif, ou électromagnétique.

Le signal reçu par le capteur de mesure utilisé comme récepteur peut être un signal électrique, ou de préférence magnétique, par exemple inductif, ou électromagnétique.

Le couplage entre plusieurs capteurs de mesure peut être, de préférence, inductif ou capacitif.

L'au moins un capteur de mesure peut comprendre une partie utilisée comme récepteur et une partie utilisée comme émetteur.

Le dispositif d'imagerie selon l'invention peut comprendre plusieurs capteurs de mesure formant une pluralité de matrices de capteurs de mesure agencées pour entourer au moins une partie du corps comprenant ledit implant, chaque matrice pouvant comprendre :
- un capteur de mesure utilisé comme émetteur, et
- une pluralité de capteurs de mesure utilisés comme récepteurs et positionnés sur le capteur de mesure utilisé comme émetteur.

Un tel agencement permet d'améliorer la précision de la mesure des signaux mesurés car les capteurs de mesure sont plus sensibles aux modifications du champ induit par les mouvements de l'implant.

Le capteur de mesure utilisé comme émetteur peut être une boucle et/ou former une boucle. La boucle est de préférence fermée et/ou de forme circulaire.

Les capteurs de mesure utilisés comme récepteurs peuvent être positionnés sur l'ensemble du pourtour du capteur de mesure utilisé comme émetteur.

Chaque matrice de capteurs de mesure est agencée pour fournir un nombre n de mesures du signal mesuré fonction du nombre de capteurs de mesure utilisés comme récepteurs dans ladite matrice.

Les capteurs de mesure de chaque boucle peuvent être couplés les uns aux autres par induction mutuelle.

Chaque capteur de mesure couplé à un autre capteur de mesure dans la matrice peut être agencé pour fournir une mesure du signal mesuré relatif audit implant.

La mesure fournie par chaque capteur de mesure couplé à un autre capteur de mesure peut comprendre une inductance mutuelle.

Au moins un capteur de mesure mesurant ledit signal mesuré peut comprendre :
- au moins un capteur capacitif, et/ou
- au moins un capteur inductif.

Chaque capteur de mesure inductif peut comprendre une bobine.

Les matrices de capteurs de mesure peuvent être similaires ou d'un agencement différent. Par exemple, le dispositif peut comprendre trois matrices donc deux similaires et une comprenant au moins un capteur de mesure en plus ou en moins par rapport aux deux autres matrices.

Le dispositif d'imagerie selon l'invention peut comprendre au moins un moyen d'enregistrement agencé pour acquérir dans le temps l'au moins un signal électrique.

Le dispositif selon l'invention peut donc réaliser une vidéo ou séquences d'images de l'implant.

L'au moins un signal électrique mesuré par le capteur ou les plusieurs capteurs de mesure peut comprendre :
- l'impédance électrique de chaque capteur de mesure, et/ou
- l'impédance électrique, dite impédance mutuelle, pour chaque capteur de mesure couplé à au moins un autre capteur de mesure, et/ou
- une tension pour chaque capteur de mesure.

La valeur de l'impédance propre et/ou mutuelle et/ou tension peut être fonction d'une orientation de l'au moins un implant.

L'unité de traitement peut être agencée pour calculer à partir de l'impédance propre ou mutuelle ou de la tension mesurée par l'au moins un capteur de mesure des angles de rotations et/ou un mouvement dudit implant.

Le dispositif d'imagerie selon l'invention peut comprendre au moins un lecteur électronique pouvant être agencé pour lire une donnée d'identification mémorisée dans un dispositif électronique intégré dans ledit implant en vue de retrouver ledit modèle visuel relatif audit implant.

Un tel dispositif électronique peut comprendre une radio-étiquette comprenant une antenne associée à une puce électronique contenant la donnée d'identification d'implant, et optionnellement, des données complémentaires.

La donnée d'identification d'implant peut comprendre l'identifiant dudit implant.

Le dispositif électronique peut comprendre le modèle visuel relatif audit implant. Ainsi, le dispositif selon l'invention peut être agencé pour lire le modèle visuel compris dans ladite radio-étiquette.

La radio-étiquette peut être un dispositif RFID, par exemple un RFID passif ou actif. La radio-étiquette peut être un transpondeur, un tag RFID.

Le dispositif électronique peut comprendre au moins un capteur agencé pour enregistrer les données complémentaires.

Le dispositif électronique, et/ou lecteur électronique et/ou l'unité de traitement selon l'invention peuvent être similaires à ceux décrits dans la demande de brevet français 3 017 227 A1 se rapportant à un dispositif de gestion de données d'implants, système comprenant ce dispositif et utilisation de ce système.

Le modèle visuel relatif audit implant peut être enregistré sur une base de données apte à communiquer avec ledit dispositif selon l'invention.

Par conséquent, le modèle visuel peut être communiqué par le dispositif électronique ou prise à partir, par exemple, du dossier médical dudit implant (dossier enregistré en réseau par exemple) enregistré sur une base de données ou un serveur local ou externe ou tout autre moyen de stockage auquel ledit dispositif selon l'invention est connecté.

Ledit implant peut comprendre au moins un indicateur de localisation.

L'indicateur de localisation peut être agencé pour donner au moins un point de repère spatial dudit implant et/ou de parties ou zones dudit implant.

L'indicateur de localisation peut comprendre au moins une protrusion et/ou une encoche dans ledit implant.

L'indicateur de localisation peut comprendre un insert composé d'un matériau différent de l'implant. Le matériau de l'insert peut être métallique, diélectrique ou magnétique.

L'indicateur de localisation peut comprendre un résonateur positionné sur ledit implant agencé pour amplifier un champ magnétique et/ou électrique à destination du ou des capteurs de mesure mesurant le signal mesuré.

Dans le contexte de l'invention, l'implant comprend au moins une partie métallique.

Le dispositif et/ou le procédé selon l'invention peuvent donc être efficaces sur des implants qui ne peuvent être imagés avec des techniques standards d'imagerie telles que l'Imagerie par Résonance Magnétique (IRM) ou les scanners.

### Brève description des dessins

D'autres avantages et particularités de l'invention apparaîtront à la lecture de la description détaillée de mises en œuvre et de modes de réalisation nullement limitatifs, et des dessins annexés suivants.
La FIGURE 1 est une représentation schématique d'un premier exemple de réalisation non limitatif d'un procédé selon l'invention ;
La FIGURE 2 est une représentation schématique d'un deuxième exemple de réalisation d'un procédé selon l'invention ;
La FIGURE 3 est un représentation schématique d'un premier exemple de réalisation non limitatif d'un dispositif selon l'invention ;
La FIGURE 4 est un représentation schématique d'un deuxième exemple de réalisation non limitatif d'un dispositif selon l'invention ;
La FIGURE 5 est un représentation schématique d'un troisième exemple de réalisation non limitatif d'un dispositif selon l'invention ;
La FIGURE 6 est une représentation schématique d'un exemple non limitatif de l'unité de traitement d'un dispositif selon l'invention.

### Description détaillée des figures

En particulier toutes les variantes et tous les modes de réalisation décrits sont combinables entre eux si rien ne s'oppose à cette combinaison sur le plan technique.

Sur les figures, les éléments communs à plusieurs figures conservent la même référence.

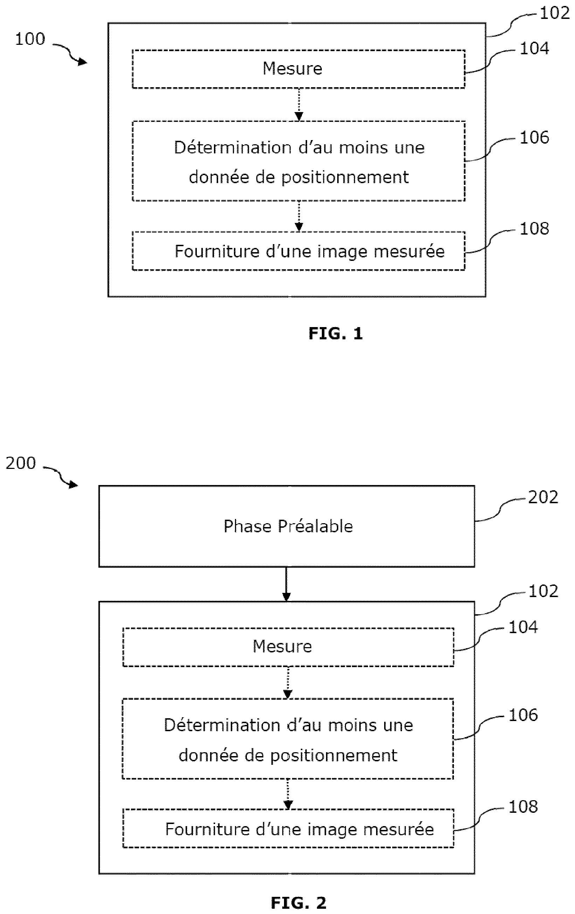

La FIGURE 1 est une représentation schématique d'un premier exemple de réalisation non limitatif d'un procédé 100 selon l'invention.

Le procédé 100 est mis en œuvre par ordinateur.

Le procédé 100 est un procédé d'imagerie 100, d'un implant implanté dans un corps, comprenant au moins une itération d'une phase de caractérisation 102.

La phase de caractérisation 102 comprend les étapes suivantes :
- mesure 104, par au moins un capteur, dit de mesure, disposé hors dudit corps, fournissant au moins un signal électrique, dit signal mesuré, relatif audit implant,
- détermination 106, par une unité de traitement, d'au moins une donnée relative au positionnement dudit implant en fonction dudit signal mesuré et d'un modèle préalablement établi reliant l'au moins un signal mesuré à l'au moins une donnée relative au positionnement, et
- fourniture 108, par une unité de traitement, d'une image, dite mesurée, à partir de l'au moins une donnée positionnement et d'un modèle visuel relatif audit implant.

De préférence, le capteur de mesure est agencé pour mesurer au moins un champ magnétique et fournir, en sortie du capteur de mesure, au moins un signal électrique.

Le modèle visuel peut être une image à l'échelle dudit implant, par exemple une image fournie par le constructeur dudit implant.

Le modèle préalablement établi peut être un réseau neuronal supervisé prenant en entrée l'au moins un signal mesuré. La base de données peut relier un signal mesuré avec au moins une donnée de positionnement.

Dans une première variante de procédé 100, le modèle visuel est une image en deux dimensions. L'image mesurée peut ainsi être en deux dimensions.

Dans une seconde variante de procédé 100, le modèle visuel est une image en trois dimensions. Dans ce cas, l'image mesurée peut être en trois dimensions ou deux dimensions.

Dans une variante du procédé 100, l'étape de mesure 104 peut comprendre une mesure d'une donnée inertielle relative au corps. Dans ce cas, l'étape de génération de l'image mesurée peut comprendre un ajustement de l'orientation de du modèle visuel.

Chaque itération de la phase de caractérisation 102 fournit une image mesurée.

Le procédé 100 peut réaliser plusieurs itérations de la phase de caractérisation 102. De cette manière, plusieurs images mesurées sont obtenues. Le procédé 100 peut, à partir de la pluralité d'images mesurées obtenues, générer une vidéo ou séquence d'images. Ainsi, les propriétés dynamiques de l'implant peuvent être observées et/ou étudiées.

La FIGURE 2 est une représentation schématique d'un deuxième exemple de réalisation d'un procédé 200 selon l'invention.

Le procédé d'imagerie 200 comprend les mêmes étapes que le procédé 100 illustré en FIGURE 1.

Le procédé 200 comprend en outre une phase préalable 202. La phase préalable 202 a été réalisée avant la première itération de la phase de caractérisation 102. En particulier, la phase préalable 202 est réalisée lorsque ledit implant est en dehors dudit corps.

La phase préalable 202 comprend une étape de mesure, par au moins un capteur, dit de mesure, disposé hors dudit corps, d'au moins un signal électrique, dit de référence.

La phase préalable 202 comprend aussi une étape de mémorisation, dudit au moins un signal de référence en association avec au moins une donnée de positionnement, dite donnée de positionnement de référence, dudit implant.

L'étape de mémorisation peut être réalisée sur un élément de stockage tel qu'un disque dur interne ou externe à l'unité de traitement, une base de données locale ou externe connectée à l'unité de traitement.

De préférence, pendant la phase préalable 202, l'implant est positionné sur un support mobile. Le support mobile comprend trois degrés de liberté en rotation, trois degrés de liberté en translation. Cela permet d'enregistrer différentes données relatives au positionnement de l'implant associées avec au moins un signal de référence. L'implant est de préférence imagé selon tous ses angles de vue.

L'au moins un capteur de mesure de la phase préalable 202 est similaire à celui utilisé pour l'étape de mesure 104.

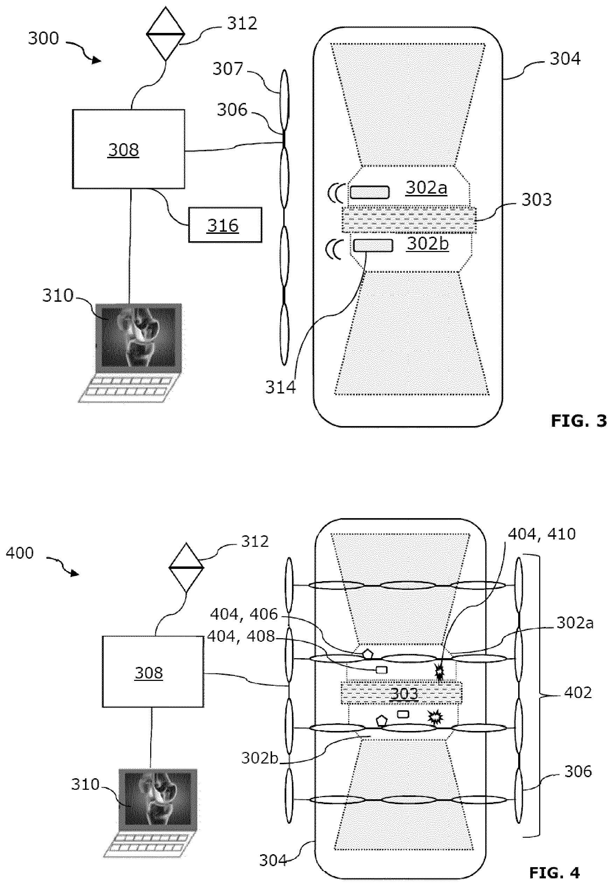

La FIGURE 3 est une représentation schématique d'un premier exemple de réalisation d'un dispositif 300 selon l'invention.

Le dispositif 300 est un dispositif d'imagerie 300 d'un implant 302 implanté dans un corps 304, agencé pour mettre en œuvre, de manière non limitative, le procédé d'imagerie 100 ou 200 illustré sur les FIGURES 1 et 2.

Le dispositif 300 comprend :
- au moins un capteur de mesure 306, disposé hors dudit corps 304 et agencé pour fournir au moins un signal mesuré relatif audit implant 302,
- l'unité de traitement 308 agencée pour :
   ∘ déterminer l'au moins une donnée relative au positionnement dudit implant 302 en fonction du signal mesuré et du modèle préalablement établi reliant l'au moins un signal mesuré à l'au moins une donnée relative au positionnement,
   ∘ fournir l'image mesurée 310, à partir de l'au moins une donnée de positionnement et du modèle visuel relatif audit implant 302.

Le signal reçu par le capteur de mesure 306 est de préférence un champ magnétique. Le signal mesuré en sortie du capteur de mesure 306 est de préférence une tension.

La FIGURE 3 illustre un implant 302 implanté dans un être vivant, en particulier un humain ou un robot. De manière non limitative, l'implant correspond à une prothèse du genou, et comprend deux parties 302a et 302b, dont une première partie 302a correspondant à un implant fémoral 302a et une deuxième partie 302b correspondant à un implant tibial 302b. Les deux parties 302a et 302b de l'implant 302 sont mobiles l'une par rapport à l'autre. L'implant 302 illustré en FIGURE 3 comprend aussi une couche intermédiaire 303 positionnée entre les deux parties 302a et 302b de l'implant 302. Bien entendu, la couche intermédiaire 303 telle que représentée en FIGURE 3 peut s'adapter à tous types d'implants articulaires, tels que les patins des prothèses de genou, cupule humérale des prothèses d'épaules, cupule cotyloïdienne des prothèses des hanches, etc.

L'implant 302 est en métal ou comprend du métal. L'implant 302 peut aussi comprendre d'autres matériaux, par exemple, du plastique, et/ou un matériau en polymère. La couche intermédiaire peut être en polyéthylène.

L'unité de traitement 308 peut être un moyen de calcul, tel qu'un processeur, un ordinateur, agencé pour exécuter un programme informatique ou des lignes de commandes dédiées à l'étape ou l'opération à réaliser.

L'unité de traitement 308 peut comprendre plusieurs modules de calcul, en particulier tous les modules, peuvent être intégrés dans un même processeur.

Le dispositif 300 comprend un capteur de mesure 306 comprenant plusieurs sites de captation 307 pour capter l'au moins un signal mesuré.

De manière optionnelle, le dispositif 300 illustré en FIGURE 3 comprend au moins un capteur inertiel 312. L'au moins un capteur inertiel 312 est agencé pour mesurer au moins une donnée inertielle relative au corps 304, ladite donnée inertielle étant utilisée par l'unité de traitement 308 pour ajuster l'orientation du modèle visuel. Dans une variante, le capteur inertiel 312 peut être au contact du corps 304.

De manière optionnelle, le capteur de mesure 306 illustré en FIGURE 3 réalisant la mesure du signal mesuré comprend au moins un capteur de champ proche. Le capteur de mesure 306 n'est pas au contact du corps 304. Dans le cas de la FIGURE 3, le capteur de mesure 306 mesurant ledit signal mesuré comprend des capteurs inductifs.

Chaque capteur de mesure 306, inductif, peut comprendre au moins une bobine (non illustrée). Le signal mesuré à la sortie de chaque capteur de mesure 306 est une tension induite sur l'au moins une bobine dudit capteur de mesure 306. Le capteur de mesure 306 est un capteur de position. En particulier, le capteur de mesure 306 peut comprendre un capteur de proximité 306, détectant de préférence des matériaux conducteurs, par exemple, le métal. Le capteur de mesure 306 permet ainsi de détecter la position de l'implant 302 ou des zones de l'implant 302. En particulier, et dans le cas illustré en FIGURE 3, le capteur de mesure 306 permet de détecter la position des deux parties 302a et 302b de l'implant 302 et de la couche intermédiaire 303.

Chaque site de captation du capteur de mesure 306 est agencé pour fournir une tension.

Chaque partie 302a, 302b de l'implant 302 comprend au moins un dispositif électronique 314. Chaque dispositif électronique 314 illustré en FIGURE 3 comprend au moins une radio-étiquette (non illustrée), ladite radio-étiquette comprenant une antenne (non illustrée) associée à une puce électronique (non illustrée) contenant une donnée d'identification d'implant. Dans le cas illustré en FIGURE 3, chaque dispositif électronique 314 comprend une donnée d'identification d'implant. La donnée d'identification d'implant comprend au moins un identifiant de l'implant 302, dans ce cas celui de la partie 302a ou 302b. De manière optionnelle, la puce électronique peut comprendre des données complémentaires. La puce électronique peut comprendre aussi un moyen de stockage pour stocker l'identifiant, et, de manière optionnelle, les données complémentaires. Les données complémentaires peuvent comprendre des données capteurs si l'implant 302 comprend au moins un capteur, par exemple un capteur de pression, température etc.

Le dispositif 300 comprend au moins un lecteur électronique 316 agencé pour échanger des données avec les dispositifs électroniques 314 positionnés respectivement dans les parties 302a et 302b de l'implant 302. En particulier, le lecteur électronique 316 est agencé pour lire la donnée d'identification d'implant mémorisée dans chaque dispositif électronique 314 en vue de retrouver le modèle visuel de chaque partie 302a et 302b de l'implant 302. La donnée d'identification d'implant de chaque partie de l'implant 302 comprend au moins l'identifiant et/ou le numéro de la partie 302a, ou 302b de l'implant 302. Cette communication est faite sans fil.

Dans le cas du dispositif 300, le modèle visuel de l'implant 302, notamment le modèle visuel de chaque partie de l'implant, est enregistré avec la donnée d'identification d'implant sur une base de données connectée à l'unité de traitement 308. Lancer une recherche sur cette base de données en entrant les données d'identification d'implant lues par le lecteur électronique 316 permet donc de retrouver le modèle visuel de chaque partie 302a, 302b de l'implant 302. Des données sur la couches intermédiaires 303 peuvent aussi être enregistrées avec le modèle visuel de chaque partie de l'implant 302. Le modèle visuel complet de l'implant 302 peut donc être obtenu, par exemple en assemblant les modèles visuels de chaque partie 302a, 302b de l'implant 302. Le modèle visuel de l'implant 302 peut ensuite être communiqué à l'unité de traitement 308 par une étape d'échange de données entre ladite base de données et ladite unité de traitement 308 afin de réaliser l'étape de fourniture 108 de l'image mesurée 310.

Les autres données complémentaires enregistrées dans chaque dispositif électronique 314 peuvent aussi être échangées.

La radio-étiquette de chaque dispositif électronique 314 peut être un RFID passif.

Le lecteur électronique 316 illustré en FIGURE 3 est connecté et disposé à l'extérieur de l'unité de traitement 308. Bien entendu, dans des variantes non illustrées, le lecteur électronique 316 peut être positionné avec l'unité de traitement 308 dans un même ensemble (i.e. même boitier).

Dans une variante, le dispositif 100 comprend au moins un moyen d'enregistrement agencé pour acquérir dans le temps l'au moins un signal mesuré. Ainsi, plusieurs images mesurées 310 peuvent être acquises dans le temps, ce qui permet de générer une séquence vidéo. Une imagerie dynamique tridimensionnelle ou bidimensionnelle de l'implant 302 ou de chaque partie 302a, 302b de l'implant 302 peut être obtenue, ce qui peut permettre de générer une vidéo du mouvement de l'implant 302. La séquence d'images obtenue peut donc être une séquence d'images imageant l'implant 302 en son ensemble ou des parties distinctes de l'implant 302.

Il est donc possible de modéliser le mouvement de l'implant 302 et aussi l'espace entre les deux parties 302a et 302b de l'implant 302 afin de vérifier le fonctionnement cinétique de l'implant 302. En cas de dysfonctionnement, cette modélisation peut permette, par exemple, de calculer le volume et la forme de la couche intermédiaire 303 qui serait susceptible d'améliorer la cinétique de l'implant 302. Cette couche intermédiaire 303 peut donc être faite sur-mesure.

La FIGURE 4 est une représentation schématique d'un deuxième exemple de réalisation d'un dispositif 400 selon l'invention.

Le dispositif 400 est un dispositif d'imagerie 400 d'un implant 302 implanté dans un corps 304, agencé pour mettre en œuvre le procédé d'imagerie 100 ou 200 illustré sur les FIGURES 1 et 2. Seules les différences avec le dispositif 300 illustré en FIGURE 2 seront représentées.

Le dispositif 400 illustré en figure 4 comprend tous les éléments du dispositif 300 illustré en FIGURE 3, mis à part les dispositifs électroniques 314 décrits en FIGURE 3.

Le dispositif 400 comprend plusieurs capteurs de mesure 306 formant une matrice 402 de capteurs de mesure 306. La matrice 402 de capteurs de mesure 306 est agencée pour entourer la partie du corps 304 comprenant l'implant 302.

Les signaux mesurés en sortie des capteurs de mesure 306 comprennent une tension.

À titre d'exemple non limitatif, la matrice 402 comprend huit colonnes de sept capteurs de mesure 306 de type inductif disposés de manière circulaire autour de l'implant 302. Les capteurs de mesure 306 sont tous similaires et sont chacun sous la forme une boucle planaire concentrique de dimensions 25 millimètres par 15 millimètres. La mesure d'inductance propre de chacun des capteurs de mesure 306 de la matrice 402 permet d'obtenir une cartographie (i.e. une matrice) formée de huit fois sept cellules ou pixels et dans laquelle chaque cellule ou pixel porte l'information d'inductance nécessaire à la fourniture de l'image mesurée 310. Ainsi, plus la matrice 402 comprend de capteurs de mesure 306, plus la cartographie obtenue est de meilleure résolution. Les valeurs d'inductance varient en fonction de la géométrie de l'implant 302. A titre d'exemple non limitatif, la cartographie peut être sous forme d'une matrice codée en niveau couleur.

L'au moins une donnée relative au positionnement de l'implant 302 est déterminée à partir de la cartographie mesurée combinée à une méthode par réseau neuronal supervisé ou une méthode de corrélation ou d'un abaque.

En outre, dans le cas illustré en FIGURE 4, l'implant 302 ne comprend pas de dispositif électronique 314. Par conséquent, le modèle visuel est récupéré sur une base de données qui peut être enregistrée sur un serveur local connecté à l'unité de traitement 308. A titre d'exemple non limitatif, le modèle visuel peut être récupéré sur le dossier du patient enregistré sur le réseau local auquel est connecté l'unité de traitement 308.

Chaque partie de l'implant 302 illustré en FIGURE 4 comprend au moins un indicateur de localisation 404. Dans le cas de la figure 4, chaque partie de l'implant 302 comprend une pluralité d'indicateurs géographiques 404. Chaque indicateur de localisation 404 est agencé pour donner au moins un point de repère spatial dudit implant et/ou de parties de l'implant 302 correspondant. Les indicateurs de localisation 404 de l'implant 302 ont une position connue et renvoient aussi des signaux connus. Ainsi, le signal mesuré à la position de cet indicateur de localisation 404 aura une forme propre ce qui permettra de positionner une partie de l'implant 302 dans l'espace et/ou de déterminer l'orientation de cette partie de l'implant 302 à partir de la cartographie obtenue.

Dans le cas illustré en FIGURE 4, chaque implant 302 comprend trois types d'indicateurs de localisation 404 différents.

Un premier type d'indicateur de localisation 404 est une encoche 406 ou une protrusion 406 dans l'implant 302. Cet indicateur comprend une forme particulière de manière à faciliter la reconnaissance de ce repère. La forme de l'encoche peut être multiple : carrée, circulaire, rectangulaire, triangulaire, pentagonale, étoile, patatoïde, etc.

Un deuxième type d'indicateur de localisation 404 est un insert 408 composé d'un matériau différent de l'implant 302 pour générer un contraste important lors de la génération de la cartographie construite à partir des capteurs de mesure 306.

Un troisième type d'indicateur de localisation 404 est un résonateur 410, de type LC, apposé sur l'implant 302 et agencé pour amplifier un champ magnétique et/ou électrique réémis vers les capteurs de mesure 306 de manière à créer un point chaud sur la cartographie.

Dans le dispositif 400, le modèle visuel est enregistré sur une base de données, par conséquent le dispositif 400 est agencé pour réaliser une étape d'échange de données, du procédé 100 ou 200, entre ladite unité de traitement 308 et ladite base de données pour réaliser l'étape de fourniture 108. Bien entendu, dans ce cas, le procédé selon l'invention peut comprendre de manière optionnelle une recherche sur une base de données du modèle visuel à partir de la donnée d'identification d'implant (i.e. identifiant de l'implant 302).

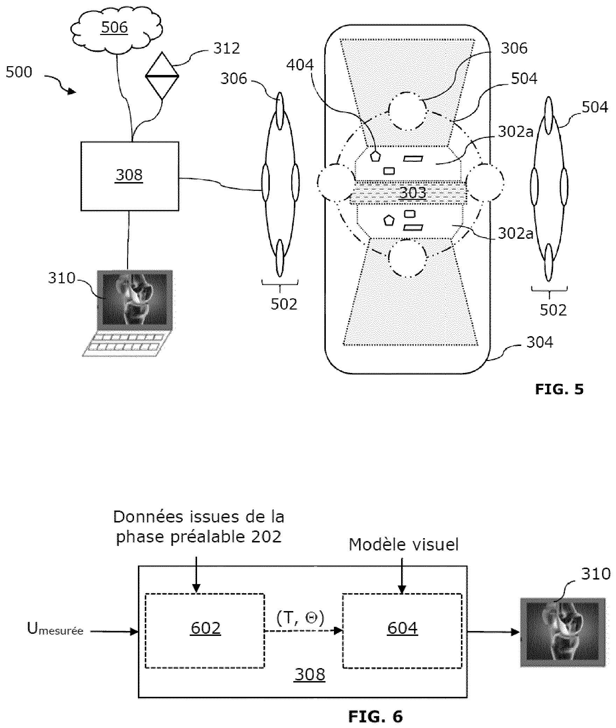

La FIGURE 5 est une représentation schématique d'un deuxième exemple de réalisation d'un dispositif 500 selon l'invention.

Le dispositif 500 est un dispositif d'imagerie 500 d'un implant 302 implanté dans un corps 304, agencé pour mettre en œuvre le procédé d'imagerie 100 ou 200 illustré sur les FIGURES 1 et 2. Seules les différences avec le dispositif 400 illustré en FIGURE 4 seront décrites.

Le dispositif 500 illustré en FIGURE 5 comprend tous les éléments du dispositif 400 illustré en FIGURE 4.

Le dispositif 500 comprend plusieurs matrices 502 de capteurs de mesure 306. En particulier, et de manière non limitative, le dispositif 500 de la FIGURE 5 comprend trois matrices 502 de capteurs de mesure 306 agencées pour entourer la partie corps 304 comprenant l'implant 302. Chaque matrice 502 comprend une pluralité de capteurs de mesure 306, dont un capteur de mesure 306 utilisé comme émetteur et plusieurs capteurs de mesure 306 utilisés comme récepteurs. Le capteur de mesure 306 utilisé comme émetteur est agencé pour émettre au moins un signal à destination de l'implant 302, et de préférence à chaque partie 302a, 302b de l'implant 302. Les capteurs de mesure 306 utilisés comme récepteurs sont agencés pour réceptionner au moins un signal provenant de chaque partie 302a, 302b de l'implant 302. Le signal réceptionné peut être un signal réfléchi (i.e. réflexion du signal émis par le capteur électrique 306 utilisé comme émetteur sur l'implant ou sur une ou chaque partie 302a, 302b de l'implant 302) par l'implant 302. Le capteur de mesure 306 utilisé comme émetteur comprend une inductance (i.e. une bobine) sous la forme d'une boucle 504. Les capteurs de mesure 306 utilisés comme récepteurs sont positionnés sur la boucle 504. Chaque capteur de mesure 306 utilisé comme récepteur sur une même boucle 504 est indépendant des autres capteurs de mesure 306 utilisés comme récepteurs sur la même boucle 504. La boucle 504 est fermée et est de forme circulaire. Les capteurs de mesure 306 utilisés comme récepteurs comprennent chacun une inductance. L'inductance peut être une bobine. Chaque matrice 502 est agencée pour fournir un nombre n de mesures du signal mesuré fonction du nombre de capteurs de mesure 306 utilisés comme récepteurs sur ladite boucle 504. Quatre capteurs de mesure 306 utilisés comme récepteurs sont positionnés sur la boucle 504 du capteur utilisé comme émetteur. Ainsi, le dispositif 500 mesure trois fois quatre signaux mesurés qui, comme dans le dispositif de la FIGURE 4, vont être représentés sous la forme d'une cartographie (i.e. matrice) de taille 3 x 4. Dans le dispositif 500 illustré en FIGURE 5, les matrices 502 sont similaires. Dans une variante non illustrée, les matrices peuvent être différentes en taille (i.e. posséder plus ou moins de capteurs de mesure 306 utilisés comme récepteurs et/ou émetteurs) et/ou en forme. L'unité de traitement 308 peut exploiter l'ensemble des signaux ou une partie des signaux mesurés.

Dans le cas de la FIGURE 5 et de manière non limitative, le signal mesuré en sortie de chaque capteur de mesure 306 utilisé comme récepteur est une tension électrique, notamment une tension électrique induite sur chaque capteur de mesure 306 utilisé comme récepteur.

Ainsi, pour chaque matrice 502, quatre mesures d'inductances mutuelles sont réalisées afin d'obtenir une cartographie plus contrastée (i.e. de meilleur sensibilité) que l'agencement des capteurs 306 illustré en FIGURE 4.

Le dispositif 500 est connecté à un réseau informatique 506 connecté à l'unité de traitement 308. Le réseau informatique 506 est lui-même connecté à une base de données comprenant le modèle visuel relatif audit implant. Le dispositif 500 est donc apte à communiquer avec un réseau externe 506 pour récupérer l'au moins le modèle visuel pour réaliser l'étape de fourniture 108 de l'image mesurée 310. Le dispositif est aussi apte à communiquer avec le réseau externe 506 pour réaliser la phase préalable 202. Cette communication est faite via internet, par WIFI.

La FIGURE 6 est une représentation schématique d'un exemple non limitatif de l'unité de traitement 308 du dispositif 300, 400, 500 ou 600.

L'unité de traitement comprend un premier module de calcul 602 configuré pour déterminer l'au moins une donnée de positionnement en fonction de l'au moins un signal mesuré U_{mesurée} et du modèle préalablement établi.

Dans un exemple non limitatif, le modèle préalablement établi peut être un réseau neuronal supervisé entrainé avec, par exemple, les données enregistrées suite à la phase préalable 202.

Dans ce cas, le premier module de calcul 602 utilise l'au moins un signal mesuré, par exemple une tension U_{mesurée} fournie par un capteur de mesure 306. L'au moins un signal mesuré peut être la cartographie d'inductance.

L'unité de traitement peut comprendre un deuxième module de calcul 604 pour fournir l'image mesurée 310 en fonction de l'au moins une donnée de positionnement Θ, T et du modèle visuel de l'implant.

L'au moins une donnée de positionnement déterminée à l'étape de détermination 106 comprend au moins une donnée relative à la position T et l'orientation Θ de l'implant 302, et dans ce cas, de chaque partie 302a et 302b de l'implant 302. L'au moins une donnée de position T et donnée d'orientation Θ sont ensuite combinées au modèle visuel de l'implant 302 (chaque partie 302a, 302b de l'implant 302 ainsi que celui de la couche intermédiaire 303) pour fournir l'image mesurée 310. Le modèle visuel de l'implant peut comprendre une image en trois dimensions de chaque partie 302a, 302b de l'implant 302.

## Revendications

1. Procédé (100, 200) d'imagerie, d'un implant (302) comprenant au moins une partie métallique, l'implant (302) étant implanté dans un corps (304), comprenant au moins une itération d'une phase de caractérisation (102) comprenant les étapes suivantes :
- mesure (104), par au moins un capteur (306), dit de mesure, disposé hors dudit corps (304), fournissant au moins un signal électrique, dit signal mesuré, relatif audit implant, l'au moins un capteur étant un capteur de proximité configuré pour détecter l'au moins une partie métallique de l'implant (302),
- détermination (106), par une unité de traitement (308), d'au moins une donnée relative au positionnement dudit implant (302) en fonction dudit signal mesuré et d'un modèle préalablement établi reliant l'au moins un signal mesuré à l'au moins une donnée relative au positionnement, l'au moins une donnée relative au positionnement comprenant au moins une donnée d'orientation de l'implant (302), et
- fourniture (108), par ladite unité de traitement (308), d'une image, dite mesurée (310), à partir de l'au moins une donnée positionnement et d'un modèle visuel relatif audit implant (302),
le procédé comprenant une étape d'adaptation du modèle visuel en fonction de l'au moins une donnée relative au positionnement de l'implant (302).

2. Procédé (100, 200) selon la revendication 1, dans lequel le modèle visuel relatif audit implant est une image tridimensionnelle dudit implant (302), et dans lequel l'image mesurée (310) est une image tridimensionnelle.

3. Procédé (100, 200) selon la revendication 1 ou 2, dans lequel l'étape de mesure (104) comprend une mesure, par au moins un capteur inertiel (312), d'au moins une donnée inertielle relative au corps (304), et dans lequel l'étape de fourniture (108) de l'image mesurée (310) comprend un ajustement de l'orientation du modèle visuel.

4. Procédé (100, 200) selon l'une quelconque des revendications précédentes, comprenant plusieurs itérations de la phase de caractérisation (102) fournissant chacune une image mesurée (310), le procédé (100, 200) comprenant en outre une génération d'une vidéo à partir desdites images mesurées (310).

5. Procédé (100, 200) selon l'une quelconque des revendications précédentes, comprenant une étape d'identification en vue de retrouver ledit modèle visuel relatif audit implant, ladite étape d'identification comprenant une étape de lecture, par un lecteur électronique (316), d'une donnée d'identification mémorisée dans un dispositif électronique (314) intégré dans ledit implant (302).

6. Procédé (100, 200) selon l'une quelconque des revendications précédentes, dans lequel le modèle visuel est enregistré sur une base de données, ledit procédé (100, 200) comprenant une étape d'échange de données entre ladite unité de traitement (308) et ladite base de données pour réaliser l'étape de fourniture (108).

7. Procédé (100, 200) selon l'une quelconque des revendications précédentes, comprenant, préalablement à la première itération de la phase de caractérisation, une phase préalable (202) réalisée lorsque ledit implant (302) est en dehors dudit corps (304), ladite phase préalable (202) comprenant au moins une itération des étapes suivantes de :
- mesure, par au moins un capteur, dit de mesure, disposé hors dudit corps (304), fournissant au moins un signal électrique, dit de référence, et
- mémorisation, sur une base de données, de l'au moins un signal de référence en association avec au moins une donnée de positionnement, dite donnée de positionnement de référence, dudit implant (302).

8. Procédé (100, 200) selon l'une quelconque des revendications précédentes, dans lequel le modèle préalablement établi comprend :
- un réseau neuronal supervisé entrainé avec une base de données et prenant en entrée l'au moins un signal mesuré, ladite base de données reliant un signal électrique avec une donnée de positionnement, ou
- une table préenregistrée associant au moins un signal électrique à au moins une donnée relative à un positionnement dudit implant, ou
- une relation mathématique reliant l'au moins un signal mesuré à l'au moins une donnée relative au positionnement.

9. Dispositif (300, 400, 500) d'imagerie d'un implant (302) comprenant au moins une partie métallique, l'implant (302) étant implanté dans un corps (304), comprenant des moyens agencés pour mettre en œuvre le procédé d'imagerie (100, 200) selon l'une quelconque des revendications précédentes, ledit dispositif (300, 400, 500) comprenant :
- au moins un capteur (306), dit de mesure, disposé hors dudit corps (304) et agencé pour fournir au moins un signal électrique, dit signal mesuré, relatif audit implant (302), l'au moins un capteur étant un capteur de proximité configuré pour détecter l'au moins une partie métallique de l'implant (302),
- une unité de traitement (308) agencée pour :
∘ déterminer au moins une donnée relative au positionnement dudit implant (302) en fonction du signal mesuré et d'un modèle préalablement établi reliant l'au moins un signal mesuré à l'au moins une donnée relative au positionnement, l'au moins une donnée relative au positionnement comprenant au moins une donnée d'orientation de l'implant (302),
∘ fournir une image (310), dite mesurée, à partir de l'au moins une donnée de positionnement et d'un modèle visuel relatif audit implant (302), et
∘ adapter le modèle visuel en fonction de l'au moins une donnée relative au positionnement de l'implant (302).

10. Dispositif (300, 400, 500) selon la revendication 9, comprenant au moins un capteur inertiel (312) agencé pour mesurer au moins une donnée inertielle relative au corps (304), ladite donnée inertielle étant utilisée par l'unité de traitement (308) pour ajuster l'orientation du modèle visuel.

11. Dispositif (300, 400, 500) selon la revendication 9 ou 10, dans lequel au moins un capteur de mesure (306) réalisant la mesure dudit signal mesuré comprend au moins un capteur de champ proche.

12. Dispositif (400) selon l'une quelconque des revendications 9 à 11, comprenant plusieurs capteurs de mesure (306) formant une matrice (402) de capteurs (306), ladite matrice (402) étant agencée pour entourer au moins une partie du corps (304) comprenant ledit implant (302).

13. Dispositif (500) selon l'une quelconque des revendications 9 à 12, comprenant plusieurs capteurs de mesure (306) formant une pluralité de matrices (502) de capteurs de mesure (306) agencées pour entourer une partie du corps (304) comprenant ledit implant (302), chaque matrice (502) comprenant :
- un capteur de mesure (306) utilisé comme émetteur, et
- une pluralité de capteurs de mesure (306) utilisés comme récepteurs et positionnés sur le capteur de mesure (306) utilisé comme émetteur.

14. Dispositif (300, 400, 500) selon l'une quelconque des revendications 9 à 13, dans lequel au moins un capteur de mesure (306) mesurant ledit signal mesuré comprend :
- au moins un capteur capacitif, et/ou
- au moins un capteur inductif.

15. Dispositif (300, 400, 500) selon l'une quelconque des revendications 9 à 14, comprenant au moins un lecteur électronique (316) agencé pour lire une donnée d'identification mémorisée dans un dispositif électronique (314) intégré dans ledit implant (302) en vue de retrouver ledit modèle visuel relatif audit implant (302).

## Patentansprüche

1. Verfahren (100, 200) zur Abbildung eines Implantats (302), das zumindest einen metallischen Abschnitt umfasst, wobei das Implantat (302) in einen Körper (304) implantiert ist, umfassend zumindest einen Durchlauf einer Charakterisierungsphase (102), die die folgenden Schritte umfasst:
- Messen (104) mittels zumindest eines Sensors (306), Messsensor genannt, der außerhalb des Körpers (304) angeordnet ist und zumindest ein elektrisches Signal, Messsignal genannt, bezüglich des Implantats bereitstellt, wobei der zumindest eine Sensor ein Näherungssensor ist, der dazu ausgelegt ist, den zumindest einen metallischen Abschnitt des Implantats (302) zu erfassen,
- Ermitteln (106), durch eine Verarbeitungseinheit (308), von zumindest einer Angabe bezüglich der Positionierung des Implantats (302) in Abhängigkeit von dem Messsignal und von einem zuvor erstellten Modell, das das zumindest eine Messsignal mit der zumindest einen Angabe bezüglich der Positionierung verknüpft, wobei die zumindest eine Angabe bezüglich der Positionierung zumindest eine Angabe zur Ausrichtung des Implantats (302) umfasst, und
- Bereitstellen (108) einer Abbildung, Messabbildung (310) genannt, durch die Verarbeitungseinheit (308), ausgehend von der zumindest einen Angabe bezüglich der Positionierung und einem visuellen Modell bezüglich des Implantats (302),
wobei das Verfahren einen Schritt der Anpassung des visuellen Modells in Abhängigkeit von der zumindest einen Angabe bezüglich der Positionierung des Implantats (302) umfasst.

2. Verfahren (100, 200) nach Anspruch 1, wobei das visuelle Modell bezüglich des Implantats eine dreidimensionale Abbildung des Implantats (302) ist und wobei die Messabbildung (310) eine dreidimensionale Abbildung ist.

3. Verfahren (100, 200) nach Anspruch 1 oder 2, wobei der Schritt des Messens (104) eine Messung zumindest einer Trägheitsangabe bezüglich des Körpers (304) durch zumindest einen Trägheitssensor (312) umfasst, und wobei der Schritt des Bereitstellens (108) der Messabbildung (310) ein Einstellen der Ausrichtung des visuellen Modells umfasst.

4. Verfahren (100, 200) nach einem der vorhergehenden Ansprüche, umfassend mehrere Durchläufe der Charakterisierungsphase (102), die jeweils eine Messabbildung (310) bereitstellen, wobei das Verfahren (100, 200) ferner das Erzeugen eines Videos aus den Messabbildungen (310) umfasst.

5. Verfahren (100, 200) nach einem der vorhergehenden Ansprüche, umfassend einen Identifikationsschritt zum Erkennen des visuellen Modells bezüglich des Implantats, wobei der Identifikationsschritt einen Schritt des Auslesens einer Identifikationsangabe, die in einer in das Implantat (302) integrierten elektronischen Vorrichtung (314) abgespeichert ist, durch ein elektronisches Lesegerät (316) umfasst.

6. Verfahren (100, 200) nach einem der vorhergehenden Ansprüche, wobei das visuelle Modell in einer Datenbank aufgezeichnet wird, wobei das Verfahren (100, 200) einen Schritt des Austauschs von Daten zwischen der Verarbeitungseinheit (308) und der Datenbank umfasst, um den Schritt des Bereitstellens (108) auszuführen.

7. Verfahren (100, 200) nach einem der vorhergehenden Ansprüche, umfassend vor dem ersten Durchlauf der Charakterisierungsphase eine Vorphase (202), die erfolgt, wenn sich das Implantat (302) außerhalb des Körpers (304) befindet, wobei die Vorphase (202) zumindest einen Durchlauf der folgenden Schritte umfasst:
- Messen durch zumindest einen Sensor, Messsensor genannt, der außerhalb des Körpers (304) angeordnet ist und zumindest ein elektrisches Signal, Referenzsignal genannt, bereitstellt, und
- Abspeichern des zumindest einen Referenzsignals in Verbindung mit zumindest einer Positionierungsangabe, Referenzpositionierungsangabe genannt, des Implantats (302) in einer Datenbank.

8. Verfahren (100, 200) nach einem der vorhergehenden Ansprüche, wobei das zuvor erstellte Modell das Folgende umfasst:
- ein überwachtes neuronales Netz, das mit einer Datenbank trainiert wird und als Eingang das zumindest eine Messsignal aufnimmt, wobei die Datenbank ein elektrisches Signal mit einer Positionierungsangabe verknüpft, oder
- eine voraufgezeichnete Tabelle, die zumindest ein elektrisches Signal mit zumindest einer Angabe bezüglich einer Positionierung des Implantats verknüpft, oder
- eine mathematische Beziehung, die das zumindest eine Messsignal mit der zumindest einen Angabe bezüglich der Positionierung verknüpft.

9. Vorrichtung (300, 400, 500) zur Abbildung eines Implantats (302), das zumindest einen metallischen Abschnitt umfasst, wobei das Implantat (302) in einen Körper (304) implantiert ist, umfassend Mittel zum Durchführen des Abbildungsverfahrens (100, 200) nach einem der vorhergehenden Ansprüche, wobei die Vorrichtung (300, 400, 500) umfasst:
- zumindest einen Sensor (306), Messsensor genannt, der außerhalb des Körpers (304) angeordnet und dazu ausgelegt ist, zumindest ein elektrisches Signal, Messsignal genannt, bezüglich des Implantats (302) bereitzustellen, wobei der zumindest eine Sensor ein Näherungssensor ist, der dazu ausgelegt ist, den zumindest einen metallischen Abschnitt des Implantats (302) zu erfassen,
- eine Verarbeitungseinheit (308), die ausgelegt ist zum
o Ermitteln zumindest einer Angabe bezüglich der Positionierung des Implantats (302) in Abhängigkeit von dem Messsignal und einem zuvor erstellten Modell, das das zumindest eine Messsignal mit der zumindest einen Angabe bezüglich der Positionierung verknüpft, wobei die zumindest eine Angabe bezüglich der Positionierung zumindest eine Angabe bezüglich der Ausrichtung des Implantats (302) umfasst,
o Bereitstellen einer Abbildung (310), Messabbildung genannt, ausgehend von der zumindest einen Positionierungsangabe und einem visuellen Modell bezüglich des Implantats (302), und
o Anpassen des visuellen Modells in Abhängigkeit von der zumindest einen Angabe bezüglich der Positionierung des Implantats (302).

10. Vorrichtung (300, 400, 500) nach Anspruch 9, umfassend zumindest einen Trägheitssensor (312), der dazu ausgelegt ist, zumindest eine Trägheitsangabe bezüglich des Körpers (304) zu messen, wobei die Trägheitsangabe von der Verarbeitungseinheit (308) verwendet wird, um die Ausrichtung des visuellen Modells einzustellen.

11. Vorrichtung (300, 400, 500) nach Anspruch 9 oder 10, wobei zumindest ein Messsensor (306), der die Messung des Messsignals durchführt, zumindest einen Nahfeldsensor umfasst.

12. Vorrichtung (400) nach einem der Ansprüche 9 bis 11, umfassend mehrere Messsensoren (306), die ein Array (402) von Sensoren (306) bilden, wobei das Array (402) dazu ausgelegt ist, zumindest einen Teil des Körpers (304), der das Implantat (302) enthält, zu umgeben.

13. Vorrichtung (500) nach einem der Ansprüche 9 bis 12, umfassend mehrere Messsensoren (306), die eine Vielzahl von Arrays (502) von Messsensoren (306) bilden, die dazu ausgelegt sind, einen Teil des Körpers (304), der das Implantat (302) enthält, zu umgeben, wobei jedes Array (502) umfasst:
- einen Messsensor (306), der als Sender verwendet wird, und
- eine Vielzahl von Messsensoren (306), die als Empfänger verwendet werden und an dem als Sender verwendeten Messsensor (306) positioniert sind.

14. Vorrichtung (300, 400, 500) nach einem der Ansprüche 9 bis 13, wobei zumindest ein Messsensor (306), der das Messsignal misst, umfasst:
- zumindest einen kapazitiven Sensor, und/oder
- zumindest einen induktiven Sensor.

15. Vorrichtung (300, 400, 500) nach einem der Ansprüche 9 bis 14, umfassend zumindest ein elektronisches Lesegerät (316), das dazu ausgelegt ist, eine Identifikationsangabe auszulesen, die in einer elektronischen Vorrichtung (314) abgespeichert ist, die in das Implantat (302) integriert ist, um das visuelle Modell bezüglich des Implantats (302) zu erkennen.

## Claims

1. A method (100, 200) for imaging an implant (302) comprising at least a metallic part, the implant (302) being implanted in a body (304), comprising at least one iteration of a characterization phase (102) comprising the following steps:
- measurement (104), by at least one sensor (306), known as the measurement sensor, located outside said body (304), supplying at least one electrical signal, known as the measured signal, relating to said implant, the at least one sensor being a proximity sensor configured to detect the at least one metallic part of the implant (302),
- determination (106), by a processing unit (308) of at least one item of data relating to the positioning of said implant (302) as a function of said measured signal and of a previously established model linking the at least one measured signal to the at least one item of data relating to positioning, the at least one item of data relating to positioning comprising at least one orientation data of the implant (302), and
- provision (108), by a processing unit (308), of an image, referred to as measured (310), from the at least one item of positioning data and a visual model relating to said implant (302),
the method comprising a step of adaptation of the visual model depending on the at least one data relating to positioning of the implant (302).

2. The method (100, 200) according to claim 1, wherein the visual model relating to said implant is a three-dimensional image of said implant (302), and wherein the measured image (310) is a three-dimensional image.

3. The method (100, 200) according to claim 1 or 2, wherein the measuring step (104) comprises measuring, by at least one inertial sensor (312), at least one item of inertial data relating to the body (304), and wherein the step of providing (108) the measured image (310) comprises an adjustment of the orientation of the visual model.

4. The method (100, 200) according to any of the preceding claims, comprising multiple iterations of the characterization phase (102) each providing a measured image (310), the method (100, 200) further comprising generating a video from said measured images (310).

5. The method (100, 200) according to any one of the preceding claims, comprising an identification step in order to retrieve said visual model relating to said implant, said identification step comprising a step of reading, by an electronic reader (316), an item of identification data stored in an electronic device (314) integrated into said implant (302).

6. The method (100, 200) according to any one of the preceding claims, wherein the visual model is stored in a database, said method (100, 200) comprising a step of exchanging data between said processing unit (308) and said database to perform the step of provision (108).

7. The method (100, 200) according to any one of the preceding claims, comprising, prior to the first iteration of the characterization phase, a preliminary phase (202) carried out when said implant (302) is outside said body (304), said preliminary phase (202) comprising at least one iteration of the following steps of:
- measurement, by at least one sensor, known as the measurement sensor, located outside said body (304), supplying at least one electrical signal, known as the reference signal, and
- storage, in a database, of the at least one reference signal in association with at least one item of positioning data, referred to as an item of reference positioning data, of said implant (302).

8. The method (100, 200) according to any one of the preceding claims, wherein the previously established model comprises:
- a supervised neural network trained with a database and taking as input the at least one measured signal, said database linking an electrical signal with an item of positioning data, or
- a pre-recorded table associating at least one electrical signal with at least one item of data relating to the positioning of said implant, or
- a mathematical relationship between the at least one measured signal and the at least one item of positioning data.

9. A device (300, 400, 500) for imaging an implant (302) comprising at least a metallic part, the implant (302) being implanted in a body (304), comprising means arranged to implement the imaging method (100, 200) according to any of the preceding claims, said device (300, 400, 500) comprising:
- at least one sensor (306), known as the measurement sensor, located outside said body (304) and arranged to supply at least one electrical signal, known as the measured signal, relating to said implant (302), the at least one sensor being a proximity sensor configured to detect the at least one metallic part of the implant (302),
- a processing unit (308) arranged to:
∘ determine at least one item of data relating to the positioning of said implant (302) as a function of the measured signal and of a previously established model linking the at least one measured signal to the at least item of data relating to positioning, the at least one item of data relating to positioning comprising at least one orientation data of the implant (302), and
∘ provide an image (310), referred to as the measured image, from at least one item of positioning data and a visual model relating to said implant (302),
∘ adapt the visual model depending on the at least one data relating to positioning of the implant (302).

10. The device (300, 400, 500) according to claim 9, comprising at least one inertial sensor (312) arranged to measure at least one item of inertial data relating to the body (304), said inertial data being used by the processing unit (308) to adjust the orientation of the visual model.

11. The device (300, 400, 500) according to claim 9 or 10, wherein at least one measurement sensor (306) performing the measurement of said measured signal comprises at least one near field sensor.

12. The device (400) according to any one of claims 9 to 11, comprising multiple measurement sensors (306) forming an array (402) of sensors (306), said array (402) being arranged to surround at least part of the body (304) comprising said implant (302).

13. The device (500) according to any of claims 9 to 12, comprising multiple measurement sensors (306) forming a plurality of arrays (502) of measurement sensors (306) arranged to surround a part of the body (304) comprising said implant (302), each array (502) comprising:
- a measurement sensor (306) used as a transmitter, and
- a plurality of measurement sensors (306) used as receivers and positioned on the measurement sensor (306) used as a transmitter.

14. The device (300, 400, 500) according to any one of claims 9 to 13, wherein at least one measurement sensor (306) measuring said measured signal comprises:
- at least one capacitive sensor, and/or
- at least one inductive sensor.

15. The device (300, 400, 500) according to any one of claims 9 to 14, comprising at least one electronic reader (316) arranged to read an item of identification data stored in an electronic device (314) integrated into said implant (302) to retrieve said visual model relating to said implant (302).
